# FASCICULE DE BREVET EUROPEEN

(11) **EP 4 422 510 B1**
(45) Date de publication et mention de la délivrance du brevet: **11.03.2026**
(21) Numéro de dépôt: 22809177.3
(22) Date de dépôt: 26.10.2022
(51) Int. Cl.: A61B 10/00, A61B 10/02, A61B 5/145

(54) **DISPOSITIF À SUCCION OCULAIRE**
OKULARE ABSAUGVORRICHTUNG
OCULAR SUCTION DEVICE

(30) Priorité: 26.10.2021 FR 2111359
(43) Date de publication de la demande: 04.09.2024
(73) Titulaire: Hôpital Fondation Adolphe de Rothschild, 75019 Paris (FR)
(72) Inventeur: GABISON, Eric, 94220 Charenton Le Pont (FR)
(74) Mandataire: Novagraaf Technologies
(86) Numéro de dépôt international: PCT/FR2022/052036
(87) Numéro de publication internationale: WO 2023/073321

(56) Documents cités:
- DE-A1- 10 164 432
- US-A- 6 048 337
- US-A1- 2012 123 297
- US-A1- 2016 235 392
- US-A1- 2020 229 972

## Description

### Domaine technique

La présente invention concerne un dispositif pour prélever un échantillon de liquide interstitiel à partir d'une muqueuse, par exemple la muqueuse buccale ou oculaire, d'un animal (*e.g*. l'homme) pour son analyse.

La présente invention trouve par exemple des applications dans le domaine de l'ophtalmologie.

### Etat de la technique

Il existe des outils de succion utilisés dans le domaine de la dermatologie pour la création d'une bulle de succion sur la peau à partir de laquelle des prélèvements de fluide interstitiel cutané peuvent être réalisés. On peut citer par exemple une capsule en acier/plexiglass possédant à la base un trou de diamètre défini (4, 8, 11 ou 14mm) et reliée à une pompe à vide, qui applique une dépression légère de 200 à 300 mmHg à la surface de la peau. Cela permet au bout d'environ 2 heures d'accumuler du fluide interstitiel cutané entre le derme et l'épiderme qui se décolle alors progressivement d'où la formation d'une bulle dite bulle de succion. On peut également citer le Cutomètre^{®} destiné à mesurer les propriétés biomécaniques des couches supérieures de l'épiderme en appliquant une pression négative (aspiration) qui déforme la peau sur laquelle il est appliqué de sorte à l'aspirer dans une cupule de mesure de la sonde avant de la relâcher à l'intérieur de la sonde. La profondeur de pénétration est déterminée par un système de mesure optique sans contact composé d'une source de lumière et d'un récepteur de lumière.

Il existe également des outils de succion qui sont utilisés dans le cadre des chirurgies de l'œil, notamment Lasik. Par exemple, on peut citer l'anneau de succion qui est placé à la surface de l'œil et qui, par phénomène de succion le long de son pourtour (dépression), va se solidariser à l'œil afin de permettre la découpe précise d'un « capot » de la cornée par un autre dispositif (microkératome) glissant sur un rail constituant la surface supérieure de l'anneau de succion. De par sa morphologie, l'anneau de succion ne peut accéder qu'au contour de l'œil mais pas à la surface de l'œil qui est couverte depuis l'extérieur *vs* zone centrale (fenêtre) accessible depuis l'extérieur.

Par ailleurs différents types de prélèvements oculaires peuvent être pratiqués à la surface de l'œil (empreinte conjonctivale), à l'intérieur de l'œil (humeur aqueuse) et de type lacrymal. Dans le cas de l'empreinte conjonctivale, il s'agit d'apposer un « buvard » à la surface de l'œil afin de recueillir des cellules épithéliales conjonctivales d'une manière non- ou très peu invasive, rapide et quasiment indolore, pour une analyse biologique des maladies de la surface oculaire. Cette apposition peut être réalisée via un instrument, notamment Eyeprim^{™} (usage unique), fonctionnant sur le principe d'une seringue : un piston actionné par le pouce pousse une membrane sur l'œil, et en relâchant le piston, cette membrane revient dans un contenant fermé et peut être ensuite prélevée en fin d'analyse à l'aide d'une pince.

La Demande de Brevet US 2016/0235392 décrit un dispositif comprenant un système de liquéfaction d'un tissu associé à un système rotatif d'abrasion dudit tissu, pour provoquer la dissolution d'un ou plusieurs composants dudit tissu et leur collecte.

### Description de l'invention

L'invention est définie dans la revendication 1.

Les inventeurs ont mis au point un nouveau dispositif de prélèvement d'un échantillon de liquide interstitiel à partir d'une muqueuse (*i.e.* membrane qui tapisse les cavités de l'organisme (tube digestif, bouche, fosses nasales, bronches, anus...) qui se raccorde avec la peau au niveau des orifices naturels, et qui est lubrifiée par la sécrétion de mucus ; *e.g*. muqueuse buccale ou conjonctive), en particulier de la muqueuse oculaire ou conjonctive, permettant, par création d'une zone de succion/aspiration à la surface de la muqueuse d'un animal, *e.g.* de la conjonctive (*i.e.* la membrane muqueuse transparente qui tapisse l'intérieur des paupières ainsi que le blanc de l'œil dans sa partie bulbaire), le prélèvement d'un échantillon de liquide interstitiel (*i.e.* le fluide intermédiaire entre le sang et la cellule dont la présence favorise l'interaction entre les deux) pour son analyse.

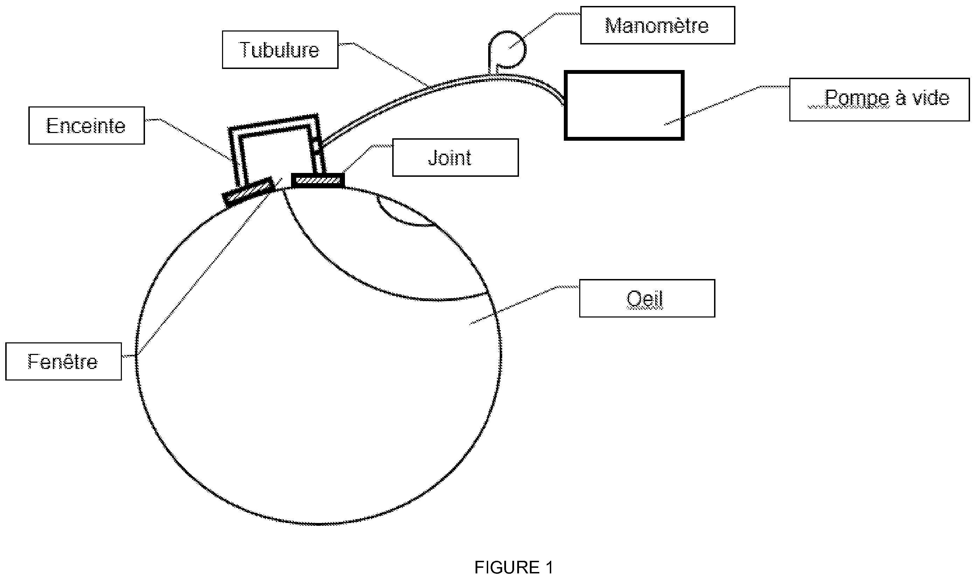

Le dispositif de l'invention comprend un volume creux, ci-après dénommé l'enceinte, relativement hermétique à l'air à l'intérieur duquel une première dépression peut être créée. De préférence, l'enceinte a des parties transparentes, notamment la face du dessus (surface distale la plus éloignée de la muqueuse, *e.g*. de la conjonctive) et du dessous (surface proximale en contact avec la muqueuse, *e.g*. la conjonctive) afin que l'opérateur puisse voir son champ opératoire. L'enceinte peut posséder éventuellement un moyen de grossissement sur le sommet de sa surface distale afin de permettre un positionnement plus précis de l'enceinte sur la surface de la muqueuse, *e.g*. de la conjonctive. La surface de l'enceinte en contact avec la muqueuse, *e.g*. la conjonctive, (surface proximale), est partiellement évidée de sorte à présenter une ouverture (« fenêtre ») définissant une zone de prélèvement. De préférence, cette surface est sensiblement courbée et bordée d'une matière, préférentiellement souple, servant de joint entre l'enceinte et la surface antérieure de la muqueuse, *e.g*. de la conjonctive, afin de s'adapter notamment à la courbure moyenne de l'œil. Il peut exister différents modèles ajustés aux différentes tailles des yeux (notamment enfant/adulte). La surface de la fenêtre, préférentiellement circulaire, peut selon les dispositifs avoir des tailles différentes comprises généralement entre 3 et 350mm². De préférence la fenêtre est construite sur une structure en iris permettant à l'opérateur, par exemple, en tournant la base de l'enceinte, de régler le diamètre de la fenêtre. Le joint bordant la surface partiellement évidée est en contact avec la muqueuse, *e.g*. la conjonctive. Afin d'aider à l'hermétisme, une crème ou toute autre matière adéquate peut être appliquée sur la muqueuse, *e.g*. la conjonctive, le long du joint. Ce joint peut se prolonger, sensiblement dans le plan de la surface partiellement évidée à l'extérieur de l'enceinte ainsi qu'à l'intérieur de l'enceinte où il peut participer à définir la zone pleine de la surface partiellement évidée. L'enceinte est reliée via une tubulure à un système permettant d'ajuster la pression de l'air dans l'enceinte. Ce système est par exemple constitué d'une pompe à vide et d'un moyen d'évaluation de la pression à l'intérieur de l'enceinte, par exemple un manomètre (cf. figure 1). D'autres systèmes connus, manuels, mécaniques, etc... peuvent aussi être utilisés pour créer une première dépression dans l'enceinte. Par exemple, un système à base d'un piston actionné manuellement, de type seringue, peut être utilisé pour créer cette dépression, tel que le corps de la seringue peut être gradué pour indiquer les niveaux de dépression générés dans l'enceinte. Le contrôle et le réglage précis et réactif de la dépression, créée progressivement dans l'enceinte est important du fait notamment de la sensibilité et la fragilité de l'œil. Le système créant la dépression dans l'enceinte peut alternativement être constitué d'un dispositif de type Vacutainer^{®}. L'intérêt d'un tel système est qu'il ne nécessite qu'un ensemble d'éléments relativement simples, notamment sans pompe à vide, qui peut alors être fabriqué sous forme de pack à usage unique. Sous ce mode, le contrôle de la dépression peut alors être réalisé à l'aide d'un système de valves reliant le Vacutainer^{®} au dispositif et/ou d'un niveau de pression dans le Vacutainer^{®} qui, rapporté au volume de l'enceinte, ne présente aucun risque pour le sujet tout en créant la dépression souhaitée. Des réglages, paliers standards, ajustements successifs, ainsi que des modes progressifs d'établissement de la dépression peuvent être définis/automatisés afin de faciliter/normaliser le travail de l'opérateur et prendre en compte le confort du sujet. Les parois de l'enceinte sont creuses et définissent un espace, en contact avec la muqueuse, *e.g*. la conjonctive, sur le pourtour de la fenêtre, à l'intérieur duquel une seconde dépression peut être créée à l'aide du premier système de dépression ou d'un système supplémentaire (*e.g*. pompe à vide, Vacutainer^{®} ou autre). Les parois de l'enceinte peuvent communiquer, ou pas, avec l'intérieur de l'enceinte. Dans le premier cas, la dépression à l'intérieur des parois de l'enceinte et à l'intérieur de l'enceinte sont identiques. La seconde dépression créée à l'intérieur des parois de l'enceinte permet de fixer la muqueuse, *e.g*. la conjonctive, le long du pourtour de la fenêtre, définissant ainsi une zone dont la partie centrale, à l'intérieur de la fenêtre, est soumise à la première dépression créée à l'intérieur de l'enceinte. On évite ainsi que la première dépression à l'intérieur de la fenêtre se traduise, du fait de la relativement grande élasticité d'une muqueuse (*e.g*. du tissu conjonctival), par un déplacement d'une trop grande quantité de muqueuse, *e.g*. de conjonctive, à l'intérieur de l'enceinte, qui risquerait d'aller jusqu'à tapisser les parois intérieures de cette dernière ; c'est-à-dire une trop grande répartition de la surpression, pour que la première dépression puisse créer un gonflement d'une partie de la muqueuse, *e.g*. de la conjonctive, *i.e*. une « bulle » de succion de liquide interstitiel qui va se former à l'intérieur de l'enceinte.

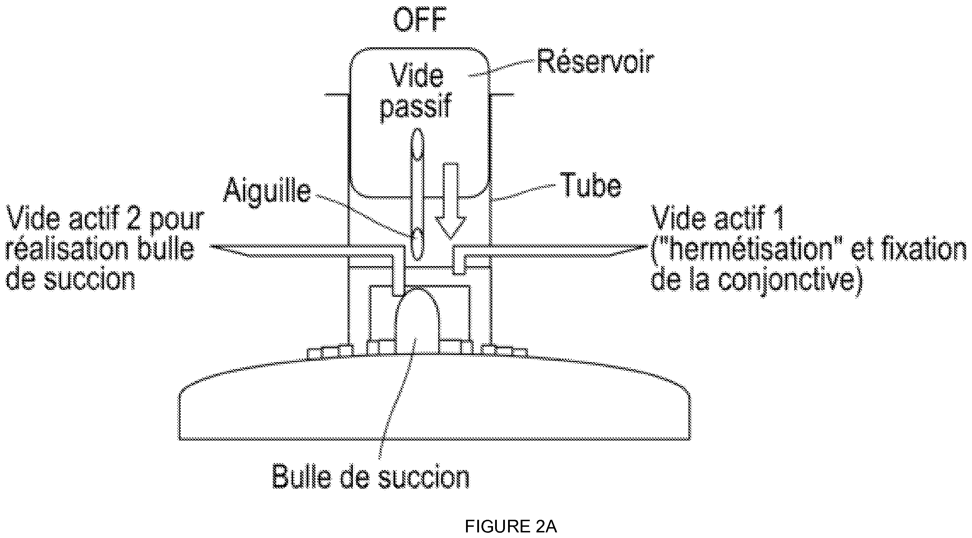

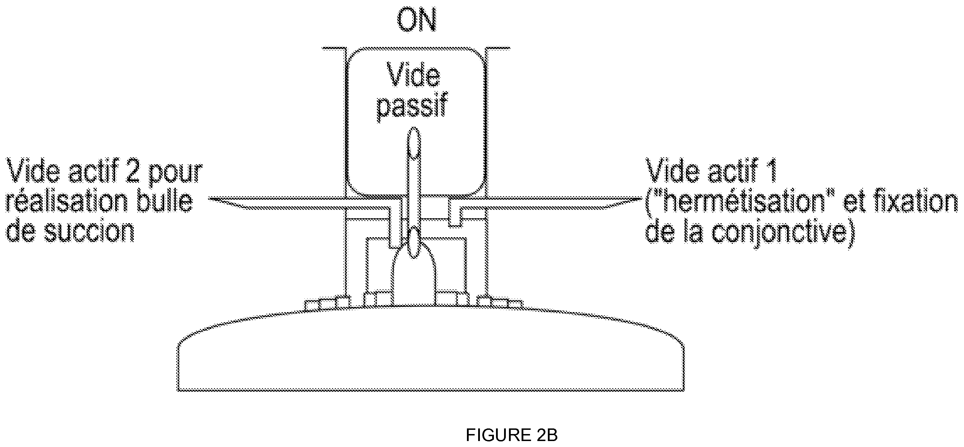

Ainsi, une fois le dispositif de l'invention appliqué à la surface de la muqueuse, e.*g*. de la conjonctive : (i) la première dépression créée à l'intérieur de l'enceinte rendue possible par l'action du système de dépression relié à l'enceinte et l'hermétisme de l'enceinte du fait du joint (non obligatoire), assure une solidarité de l'enceinte avec la muqueuse, e.*g*. la conjonctive, ainsi qu'une mise en surpression relative de la partie de la muqueuse, e.*g*. de la conjonctive, en contact avec le dispositif, en particulier celle au niveau de la fenêtre pour la formation d'une « bulle » de liquide interstitiel, et (ii) la seconde dépression créée à l'intérieur des parois de l'enceinte assure de la rendre complètement hermétique et de fixer la muqueuse, e.*g*. la conjonctive, afin de la solliciter sur une surface inférieure à la surface du pourtour du dispositif d'où un accroissement de pression localisée qui permet, sans risque de surpression (intra-oculaire dans le cas de la conjonctive), de diminuer significativement le temps nécessaire à la formation de la « bulle ». Cette mise en surpression relative/locale par le dispositif de l'invention crée donc progressivement, au niveau de la partie de la muqueuse, e.*g*. de la conjonctive, à l'intérieur de l'enceinte une « bulle » dont la surface externe est la muqueuse, e.*g*. la conjonctive, et l'intérieur le liquide interstitiel qui peut alors être prélevé. Le dispositif possède à cet effet un moyen pour effectuer un prélèvement au niveau de la partie de la muqueuse, e.*g*. de la conjonctive, accessible via la fenêtre, et notamment de profiter de l'accessibilité du liquide interstitiel dans la « bulle » du fait de la surpression relative/locale. Le prélèvement du liquide interstitiel dans la « bulle » est effectué de préférence par un phénomène de succion supplémentaire réalisé au niveau de la fenêtre par un système fonctionnant par exemple sur le principe d'un Vacutainer^{®}. Sous ce principe, le dispositif comprend une aiguille creuse capable de traverser le sommet de l'enceinte, pointant vers la fenêtre et telle qu'à l'intérieur de ladite aiguille se trouve une valve/clapet à sens unique faisant qu'un gaz/liquide ne peut passer dans l'aiguille que dans le sens de l'intérieur de l'enceinte vers l'extérieur de l'enceinte. Un tel clapet empêche également l'air extérieur d'entrer dans le contenant lors de la génération de la dépression dans l'enceinte, et permet réciproquement le passage d'élément prélevé à l'aide de l'aiguille vers l'extérieur de l'enceinte. Cette aiguille possède deux extrémités aiguisées et peut être déplacée suivant son axe sans remettre en cause l'hermétisme de sa liaison avec l'enceinte. L'aiguille peut ainsi être rapprochée de la partie de la muqueuse, e.*g*. de la conjonctive, (bulle de succion) passant par la fenêtre, voire pénétrer cette partie à la profondeur souhaitée par l'opérateur. L'autre extrémité de l'aiguille peut alors, à la manière d'un système Vacutainer^{®}, être utilisée pour percer un réservoir placé en dépression tel que la pression interne dudit réservoir est inférieure à la pression à l'intérieur de l'enceinte, créant un phénomène d'aspiration du prélèvement vers le réservoir. Pour ce faire, l'enceinte a en son sommet (surface distale) une zone, par exemple en mousse / élastomère, facilement transperçable par une aiguille similaire à celle indiquée supra, de sorte qu'elle se referme autour de l'aiguille sans laisser passer d'air aux pressions de travail du dispositif de l'invention. Un moyen pour effectuer un prélèvement peut être constitué d'un tube abouté au sommet de l'enceinte, à l'intérieur duquel peut coulisser un réservoir traversé d'une aiguille. Ce tube est hermétique et est tel qu'au repos (off) l'aiguille ne traverse pas le sommet de l'enceinte. La pression du réservoir et du tube en situation de repos est telle que, lorsqu'en faisant coulisser le réservoir vers l'enceinte (on), et qu'ainsi l'aiguille traverse le sommet de l'enceinte et par suite la muqueuse, e.*g*. la conjonctive, formant la surface extérieure de la « bulle » de succion, alors la pression du réservoir est égale à Pr, inférieure à la pression Pe à l'intérieur de l'enceinte, ce qui crée un mouvement du liquide interstitiel qui est dans la « bulle » de succion, vers l'intérieur du réservoir. Cela suppose que lorsque la « bulle » de succion est créée, le sommet de cette dernière est très proche, voire en contact avec le sommet de l'enceinte, afin de limiter la dépression générée par l'aiguille lorsque cette dernière est à l'intérieur de l'enceinte mais pas encore à l'intérieur de la « bulle » de succion (cf. figure 2A). L'ensemble du dispositif, à l'exception du ou des systèmes créant la dépression, est à usage unique. L'opérateur peut avoir autant d'aiguilles que de réservoirs, et procéder comme indiqué supra (percement du sommet de l'enceinte, rapprochement de la pointe de l'aiguille vers la partie de la muqueuse, e.*g*. de la conjonctive, passant au travers de la fenêtre, percement du réservoir pour effectuer le prélèvement de liquide interstitiel). L'intérêt de ce mode de prélèvement tient en ce qu'il n'y a pas de contamination d'un réservoir à l'autre du fait d'un partage d'aiguille au cas où l'opérateur souhaite réaliser plusieurs prélèvements (*e.g*. à des profondeurs différentes). D'autres systèmes de « perforation/aspiration » connus sont aussi envisageables pour la réalisation du prélèvement *(e.g*. seringue, etc...). Pour un tel dispositif de prélèvement, la longueur du trajet du système de perforation/aspiration (aiguille) est par sécurité préférentiellement telle que la pointe ne puisse pas aller au-delà de la partie inférieure du dispositif et donc de la protubérance formée par la bulle de succion. Par exemple, dans le cas d'une aiguille traversant un réservoir coulissant, la longueur de l'aiguille de prélèvement au sortir du réservoir est par sécurité préférentiellement inférieure à la hauteur des parois de l'enceinte bloquant le trajet du réservoir.

Le prélèvement de liquide interstitiel réalisé via le dispositif de l'invention permet des analyses inédites : conduite d'examens biochimiques (*e.g*. pH, osmolarité et ionogramme) ; recherche de cellules inflammatoires, étude pharmacocinétique/dynamique par exemple sur des collyres ou des molécules administrées par voie systémique (tant surface que profondeur) ; effets secondaires des chimiothérapies (jusqu'alors étude des larmes). Il rend également possible des analyses immédiates de type « Lab on a Chip » qui permettent d'étudier la concentration en différentes protéines du liquide interstitiel. Le dispositif de l'invention peut également permettre de profiter de la force de liaison créée par la succion pour réaliser, sur la zone en contact avec la surface de la muqueuse, e.*g*. de la conjonctive, un prélèvement de tissus de type empreinte conjonctivale ou épithéliale (*e.g*. buccale) de pleine épaisseur de la muqueuse. D'autre part, l'espace créé par le dispositif de l'invention notamment sous la conjonctive peut permettre des injections de substances dans un espace plus limité que les injections sous-conjonctivales actuelles.

La présente invention a donc pour objet un dispositif pour prélever un échantillon de liquide interstitiel à partir de la muqueuse d'un animal pour son analyse, ledit dispositif comprenant :
(i) une enceinte hermétique à l'air dont la surface proximale en contact direct avec la muqueuse présente une ouverture définissant une zone de prélèvement, et dont la surface distale comprend au moins un orifice connecté à un premier système d'aspiration (pour créer une dépression à l'intérieur de l'enceinte hermétique et la formation d'une bulle de succion à travers l'ouverture), et
(ii) un système de prélèvement constitué d'un tube abouté au sommet de la surface distale de l'enceinte hermétique, à l'intérieur duquel coulisse un réservoir traversé d'un système de perforation orienté vers la muqueuse, au regard de l'ouverture ;
dans lequel les parois de l'enceinte hermétique sont creuses (pour favoriser la fixation à la surface de la muqueuse et accroitre la pression localisée pour une formation plus rapide de la bulle de succion et de ce fait une production plus rapide de liquide interstitiel tout en limitant le risque de surpression de la muqueuse (notamment de la conjonctive dans le cas de l'œil) et connectées à un second système d'aspiration.

Selon un mode de réalisation particulier du dispositif de l'invention, la muqueuse est la muqueuse buccale ou la conjonctive, de préférence la conjonctive.

Selon un mode de réalisation particulier du dispositif de la présente invention, la surface proximale de l'enceinte est bombée.

Selon un mode de réalisation particulier du dispositif de la présente invention, tout ou partie de la surface proximale de l'enceinte est recouverte d'un joint souple d'étanchéité. En outre, la partie du dispositif en contact avec la surface de la muqueuse, e.g. de la conjonctive, peut être recouverte d'une membrane permettant d'effectuer un prélèvement de type empreinte épithéliale (*e.g*. buccale) ou conjonctivale.

Selon un mode de réalisation particulier du dispositif de la présente invention, l'ouverture (ou fenêtre) dans la surface proximale de l'enceinte a une surface comprise entre 3 et 350 mm², et est éventuellement ajustable.

Selon un mode de réalisation particulier du dispositif de la présente invention, le premier système d'aspiration, et éventuellement le second système d'aspiration, crée une dépression inférieure à environ 400 mbar (300 mmHg)

Selon un mode de réalisation particulier du dispositif de la présente invention, la surface distale de l'enceinte hermétique est recouverte au moins sur son sommet d'une matière transperçable par le système de perforation et se refermant sur ce dernier lorsque transpercé pour maintenir l'étanchéité.

Selon un mode de réalisation particulier du dispositif de la présente invention, l'enceinte hermétique a des surfaces transparentes, et éventuellement un moyen de grossissement sur le sommet de sa surface distale.

Selon un mode de réalisation particulier du dispositif de la présente invention, le réservoir a une contenance allant de 10 à 300µl, de préférence de 20 à 200µl, préférentiellement de 30 à 100µl, tout préférentiellement de 40 à 50µl ».

Selon un mode de réalisation particulier du dispositif de la présente invention, la longueur du système de perforation sortant du réservoir est inférieure à la hauteur maximale de l'enceinte hermétique.

Un procédé non revendiqué est décrit, le procédé étant utile pour comprendre l'invention. Le procédé se réfère à un procédé pour prélever un échantillon de liquide interstitiel à partir de la muqueuse d'un animal pour son analyse, ledit procédé comprenant :
- la disposition d'un dispositif selon la présente invention sur la surface de la muqueuse à l'endroit du prélèvement ;
- l'application d'une dépression dans les parois et à l'intérieur de l'enceinte hermétique de sorte à former une bulle de succion au niveau de l'ouverture de la surface proximale de l'enceinte ;
- le déplacement du réservoir du système de prélèvement en direction de la muqueuse jusqu'à ce que le système de perforation traverse la surface distale de l'enceinte hermétique et la bulle de succion ;
- la collecte de l'échantillon de liquide interstitiel de la bulle de succion dans le réservoir.

Selon un mode de réalisation particulier du dispositif de l'invention, la muqueuse est la muqueuse buccale ou la conjonctive, de préférence la conjonctive. Selon un mode de réalisation particulier du procédé utile pour comprendre l'invention, la dépression appliquée dans les parois et à l'intérieur de l'enceinte hermétique est inférieure à 400 mbar (300 mmHg de mercure).

Selon un mode de réalisation particulier du procédé utile pour comprendre l'invention, le système de perforation ne peut descendre au-delà de la surface proximale de l'enceinte hermétique.

### BREVE DESCRIPTION DES FIGURES

La Figure 1 représente l'enceinte en vue de coupe, posée sur un œil, et relié à une pompe à vide et un manomètre. La fenêtre est une ouverture sur la face de l'enceinte (surface proximale) en contact avec la surface de l'œil (conjonctive).
La Figure 2 représente un mode de prélèvement de liquide interstitiel au niveau de la surface conjonctivale de l'œil passant au travers de la fenêtre (bulle) par un dispositif de l'invention, au repos (figure 2A, off) et en action (figure 2B, on).
La Figure 3 représente le principe général de l'enceinte du dispositif de l'invention.
La Figure 4 représente la séquence d'utilisation d'un dispositif selon l'invention.

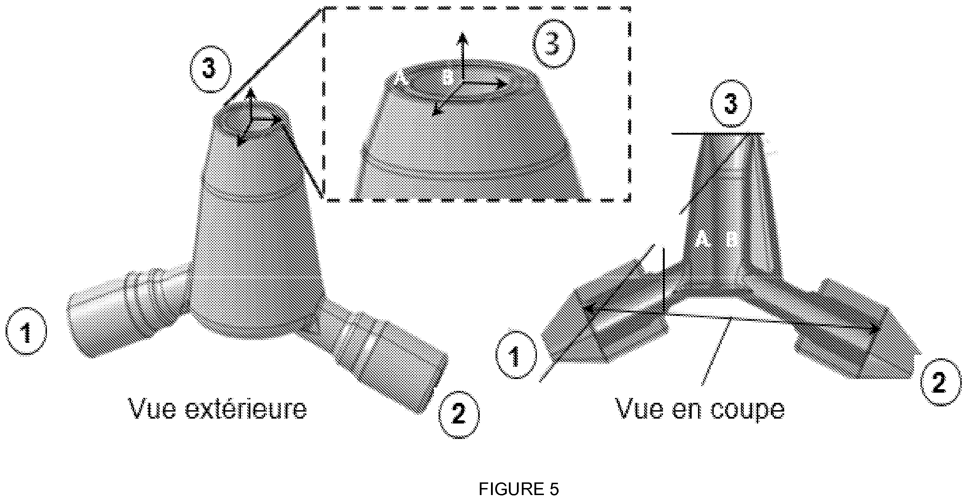

La Figure 5 représente un prototype de cloche à succion oculaire sans le dispositif de prélèvement de liquide interstitiel. (1) orifice vers le système d'aspiration du cylindre extérieur (3A) de maintien à la surface muqueuse. (2) orifice vers le système d'aspiration du cylindre intérieur (3B) pour un prélèvement de liquide interstitiel ou un prélèvement épithélial muqueux.

### EXEMPLES

### EXEMPLE 1 : EXEMPLES DE REALISATION D'UN DISPOSITIF DE L'INVENTION ET SA MISE EN OEUVRE

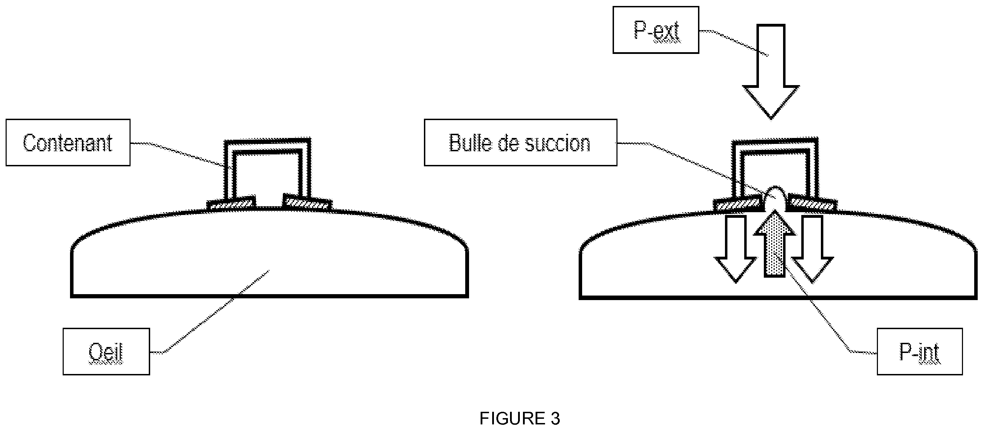

### Principe général de l'enceinte du dispositif de l'invention en action sur la conjonctive (Figure 3) :

A gauche de la figure 3, l'enceinte du dispositif de l'invention est appliquée sur l'œil.

A droite de la figure 3, la dépression générée à l'intérieur de l'enceinte a provoqué une « bulle » de succion / excroissance de l'œil au travers de la fenêtre, c'est-à-dire une bulle dont la surface est le tissu conjonctival et 'intérieur le liquide interstitiel.

La dépression à l'intérieur de l'enceinte va créer du fait de la différence de pression avec l'extérieur, une force (Pext) appliquée sur l'enceinte en direction de l'œil au niveau des zones de contact (joint) de l'enceinte avec la surface de l'œil, et symétriquement, il va y avoir une surpression (Pint) de l'intérieur de l'œil au niveau de la fenêtre poussant vers l'intérieur de l'enceinte. La combinaison de ces deux forces contraires va générer l'excroissance de l'œil (*i.e.* une bulle dont la surface extérieure est le tissu conjonctivale et l'intérieur le liquide interstitiel) au travers de la fenêtre.

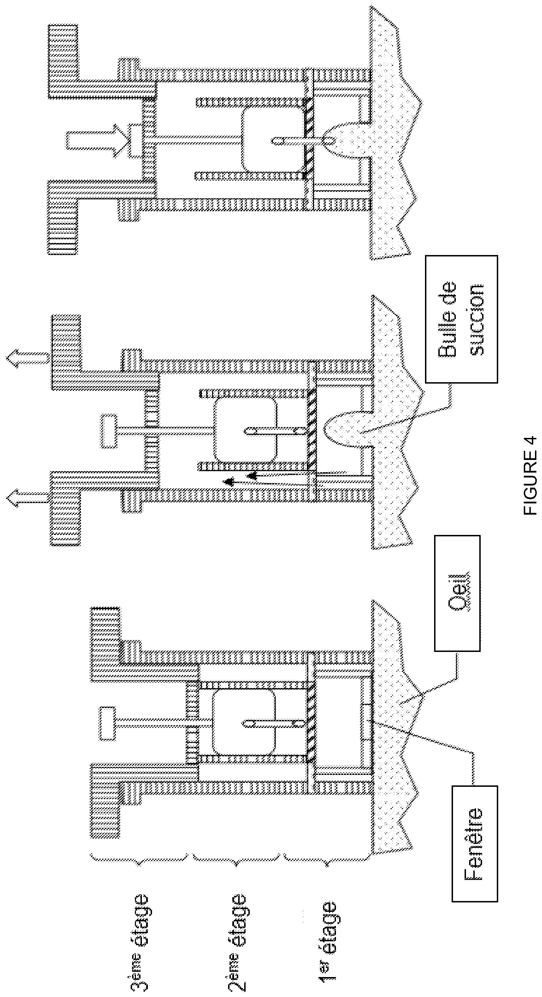

### Illustration de l'usage du dispositif de l'invention sur la conjonctive (Figure 4) :

Le moyen de prélèvement, le moyen de dépression et l'enceinte font partie du dispositif de l'invention. Ce dernier comprend un corps tubulaire à 3 étages, ayant des parois creuses. Sa partie inférieure, définissant le 1^{er} étage, est appliquée sur la conjonctive, et a une face supérieure comprenant une partie centrale hermétique et une partie périphérique communiquant avec les parois du 2^{ème} étage. Ce 2^{ème} étage comprend des parois creuses d'une épaisseur supérieure aux parois du 1^{er} étage communiquant avec les parois et l'intérieur du 1^{er} étage. Le centre de ce 2^{ème} étage possède un réservoir tubulaire pouvant coulisser de manière hermétique le long des parois, traversé sur sa face inférieure par une aiguille et prolongé en haut par un bras. La pression du 2^{ème} étage est P2 < pression atmosphérique. Le 3^{ème} étage comprend des conduits communiquant avec les parois du 2^{ème} étage et, à son sommet, une lamelle étanche coulissante, traversée en son centre par le bras issu du réservoir sans que l'air puisse passer à cet endroit.

La séquence d'utilisation de ce dispositif est la suivante : après avoir appliqué le bas du dispositif (surface proximale) sur la conjonctive, la lamelle étanche du 3^{ème} étage est coulissée de manière à créer une dépression dans les parois et à l'intérieur du 1^{er} étage (via la communication d'air entre les parois des 1^{er} et 2^{ème} étages et les conduits du 3^{ème} étage). Cette dépression P1, crée une « bulle » de succion à la surface de l'œil et est telle que P2<P1. Le bras relié au réservoir est alors appuyé, de manière à ce que l'aiguille prolongeant ce dernier traverse la face supérieure (surface distale) du 1^{er} étage et perce la « bulle » de succion, provoquant le prélèvement de liquide interstitiel. Une fois que le prélèvement a eu lieu, le retrait du dispositif peut être effectué en rabaissant le 3^{ème} étage, ce qui annule la dépression.

A gauche de la figure 4, le dispositif de l'invention est apposé sur l'œil. Le 1^{er} étage servant d'enceinte a des parois creuses et une fenêtre.

Au milieu de la figure 4, l'élévation du 3^{ème} étage crée, via un réseau de parois creuses et de conduits, une dépression dans le 1^{er} étage (intérieur et parois) qui sert d'enceinte. Cette élévation peut être rendue aisée via des anses servant de support aux doigts de l'opérateur qui de l'autre main tient le corps du dispositif. Il y a alors formation de la bulle de succion.

A droite de la figure 4, la pression sur le bras pousse le réservoir et l'aiguille au travers de la bulle de succion. Du fait de la différence de pression entre le 1^{er} étage et le réservoir, il y a aspiration du liquide interstitiel contenu dans la bulle de succion vers le réservoir.

## Revendications

1. Dispositif pour prélever un échantillon de liquide interstitiel à partir de la muqueuse d'un animal pour son analyse, ledit dispositif comprenant:
(i) une enceinte hermétique à l'air dont la surface proximale en contact direct avec la muqueuse présente une ouverture définissant une zone de prélèvement, et dont la surface distale comprend au moins un orifice connecté à un premier système d'aspiration, et
(ii) un système de prélèvement constitué d'un tube abouté au sommet de la surface distale de l'enceinte hermétique, à l'intérieur duquel coulisse un réservoir traversé d'un système de perforation orienté vers la muqueuse, au regard de l'ouverture ;
dans lequel les parois de l'enceinte hermétique sont creuses, et connectées à un second système d'aspiration.

2. Dispositif selon la revendication 1, où la muqueuse est la conjonctive.

3. Dispositif selon la revendication 1 ou 2, dans lequel la surface proximale de l'enceinte est bombée.

4. Dispositif selon l'une quelconque des revendications 1 à 3, dans lequel la surface proximale de l'enceinte est recouverte d'un joint souple d'étanchéité.

5. Dispositif selon l'une quelconque des revendications 1 à 4, dans lequel l'ouverture dans la surface proximale de l'enceinte a une surface comprise entre 3 et 350 mm², et éventuellement ajustable.

6. Dispositif selon l'une quelconque des revendications 1 à 5, dans lequel le système d'aspiration crée une dépression inférieure à 400 mbar (300 mmHg).

7. Dispositif selon l'une quelconque des revendications 1 à 6, dans lequel la surface distale de l'enceinte hermétique est recouverte au moins sur son sommet d'une matière transperçable par le système de perforation et se refermant sur ce dernier lorsque transpercé pour maintenir l'étanchéité.

8. Dispositif selon l'une quelconque des revendications 1 à 7, dans lequel l'enceinte hermétique a des surfaces transparentes, et éventuellement un moyen de grossissement sur le sommet de sa surface distale.

9. Dispositif selon l'une quelconque des revendications 1 à 8, dans lequel le réservoir a une contenance allant de 10 à 300 µl

10. Dispositif selon l'une quelconque des revendications 1 à 9, dans lequel la longueur du système de perforation sortant du réservoir est inférieure à la hauteur maximale de l'enceinte hermétique.

## Patentansprüche

1. Vorrichtung zum Entnehmen einer Probe von Interstitialflüssigkeit aus der Schleimhaut eines Tieres zur Analyse, wobei die Vorrichtung umfasst:
(i) ein luftdichtes Gehäuse, dessen proximale Oberfläche, die in direktem Kontakt mit der Schleimhaut steht, eine Öffnung aufweist, die einen Sammelbereich definiert, und dessen distale Oberfläche mindestens einen Anschluss umfasst, der mit einem ersten Absaugsystem verbunden ist, und
(ii) ein Sammelsystem, bestehend aus einem Rohr, das mit der Oberseite der distalen Oberfläche des versiegelten Gehäuses verbunden ist und in dessen Innerem ein Reservoir gleitet, das von einem zur Schleimhaut gerichteten Perforationssystem durchzogen ist, das der Öffnung gegenüberliegt;
wobei die Wände des versiegelten Gehäuses hohl sind und mit einem zweiten Absaugsystem verbunden sind.

2. Vorrichtung nach Anspruch 1, wobei die Schleimhaut die Bindehaut ist.

3. Vorrichtung nach Anspruch 1 oder 2, wobei die proximale Oberfläche des Gehäuses gewölbt ist.

4. Vorrichtung nach einem der Ansprüche 1 bis 3, wobei die proximale Oberfläche des Gehäuses mit einer flexiblen Dichtung abgedeckt ist.

5. Vorrichtung nach einem der Ansprüche 1 bis 4, wobei die Öffnung in der proximalen Oberfläche des Gehäuses eine Fläche zwischen 3 und 350 ^{mm²} hat und optional einstellbar ist.

6. Vorrichtung nach einem der Ansprüche 1 bis 5, wobei das Absaugsystem ein Vakuum von weniger als 400 mbar (300 mmHg) erzeugt.

7. Vorrichtung nach einem der Ansprüche 1 bis 6, wobei die distale Oberfläche des versiegelten Gehäuses zumindest an ihrer Oberseite mit einem Material bedeckt ist, das durch das Perforationssystem durchstochen werden kann und sich nach dem Durchstechen über diesem schließt, um die Versiegelung aufrechtzuerhalten.

8. Vorrichtung nach einem der Ansprüche 1 bis 7, wobei das versiegelte Gehäuse transparente Oberflächen und optional eine Vergrößerungseinrichtung auf der Oberseite seiner distalen Oberfläche aufweist.

9. Vorrichtung nach einem der Ansprüche 1 bis 8, wobei das Reservoir ein Fassungsvermögen im Bereich von 10 bis 300 µl aufweist.

10. Vorrichtung nach einem der Ansprüche 1 bis 9, wobei die Länge des aus dem Reservoir herausragenden Perforationssystems geringer ist als die maximale Höhe des versiegelten Gehäuses.

## Claims

1. A device for collecting a sample of interstitial fluid from the mucosa of an animal for analysis, said device comprising:
(i) an airtight enclosure whose proximal surface in direct contact with the mucosa has an opening defining a collection area, and whose distal surface comprises at least one port connected to a first suction system, and
(ii) a collection system consisting of a tube joined to the top of the distal surface of the sealed enclosure, inside which a reservoir slides that is passed through by a perforation system oriented toward the mucosa, opposite the opening;
wherein the walls of the sealed enclosure are hollow, and connected to a second suction system.

2. The device according to claim 1, where the mucosa is the conjunctiva.

3. The device according to claim 1 or 2, wherein the proximal surface of the enclosure is domed.

4. The device according to any one of claims 1 to 3, wherein the proximal surface of the enclosure is covered by a flexible seal.

5. The device according to any one of claims 1 to 4, wherein the opening in the proximal surface of the enclosure has an area of between 3 and 350 mm², and is optionally adjustable.

6. The device according to any one of claims 1 to 5, wherein the suction system creates a vacuum of less than 400 mbar (300 mmHg).

7. The device according to any one of claims 1 to 6, wherein the distal surface of the sealed enclosure is covered at least on its top with a material that can be pierced by the perforation system and closes over the latter when pierced to maintain the seal.

8. The device according to any one of claims 1 to 7, wherein the sealed enclosure has transparent surfaces, and optionally a magnification means on the top of its distal surface.

9. The device according to any one of claims 1 to 8, wherein the reservoir has a capacity ranging from 10 to 300 µl.

10. The device according to any one of claims 1 to 9, wherein the length of the perforation system emerging from the reservoir is less than the maximum height of the sealed enclosure.
